# EUROPÄISCHE PATENTSCHRIFT

(11) **EP 3 291 774 B1**
(45) Veröffentlichungstag und Bekanntmachung des Hinweises auf die Patenterteilung: **12.02.2020**
(21) Anmeldenummer: 16726017.3
(22) Anmeldetag: 06.05.2016
(51) Int. Cl.: A61F 2/954, A61F 2/958, A61M 25/10

(54) **DOPPELBALLON**
DOUBLE BALLOON
BALLONNET DOUBLE

(30) Priorität: 06.05.2015 DE 102015107038
(43) Veröffentlichungstag der Anmeldung: 14.03.2018
(73) Patentinhaber: Bentley InnoMed GmbH, 72379 Hechingen (DE)
(72) Erfinder: BREGULLA, Rainer, 72336 Balingen (DE); OBRADOVIC, Milisav, 79539 Lörrach (DE)
(74) Vertreter: Hoefer & Partner Patentanwälte mbB
(86) Internationale Anmeldenummer: PCT/EP2016/060200
(87) Internationale Veröffentlichungsnummer: WO 2016/177885

(56) Entgegenhaltungen:
- EP-A1- 2 848 279
- EP-A2- 0 835 673
- WO-A1-96/13298
- WO-A1-97/17101
- WO-A1-2006/116495
- US-A- 5 049 132
- US-A1- 2013 060 316
- US-A1- 2013 338 761

## Beschreibung

Die Erfindung betrifft einen Ballonkatheter, insbesondere für die Aufweitung von Stents in Fenestrierungen, mit einem ersten, inneren Ballon, einem zweiten äußeren Ballon, separaten Zuleitungen im Katheter zum ersten und zum zweiten Ballon, die es erlauben, die Ballone unabhängig voneinander mit Druck zu beaufschlagen, einem zentralen Lumen für einen Führungsdraht, wobei der zweite Ballon den ersten Ballon vollständig umschließt.

Ballonkatheter werden seit vielen Jahren zum Aufweiten von Stents in Gefäßen eingesetzt. Zur Aufweitung von Stents wird ein Stent auf den Ballonkatheter aufgekrimpt und mit Hilfe des Ballonkatheters am gewünschten Implantationsort dilatiert und platziert. Anschließend wird der Ballonkatheter ohne den Stent aus dem Gefäß entfernt.

Bei der Angioplastie werden Ballonkatheter eingesetzt, um ein verengtes Gefäß mechanisch aufzuweiten und dort gebildete Plaques an die Gefäßwand zu drücken.

Ein besonderes Problem tritt auf, wenn im Bereich von Gefäßabzweigungen neben dem Hauptast auch die Abzweigung mit einem Stent versehen werden muss. In diesem Fall wird zunächst ein mit einer Fenestrierung versehener Stent in den Hauptast verbracht und dort so implantiert, dass das Fenster im Bereich der Abzweigung angeordnet ist. Sodann wird ein weiterer Stent in die Abzweigung eingebracht, dort dilatiert und durch Aufweiten an den Stent im Hauptast angepasst. Dies fordert in der Regel mehrere separate Schritte, insbesondere dann, wenn sich das abzweigende Gefäß in seinem Verlauf verengt und damit eine abgestufte Aufweitung vorgenommen werden muss. Hinzu kommt die Anpassung des Stents im Seitenast an das Fenster und den Verlauf des Stents im Hauptast.

Für diese Anpassung ist es möglich, abgestuft mit mehreren Ballonen unterschiedlichen Durchmessers vorzugehen. Eingesetzt wird aber auch ein sogenannter "Ballon im Ballon", bei dem zwei Ballone so aneinander gekoppelt sind, dass sie separat mit Druck beaufschlagt und für die unterschiedliche Aufweitung eingesetzt werden können. Nachteilig ist der Aufwand beim Einsatz mehrerer separater Ballone bzw. die schlechte Anpassbarkeit der aneinander anschließenden Ballone.

Ein Ballonkatheter gemäß Oberbegriff des Anspruchs 1 ist in der WO 96/13298 A1 beschrieben.

Aufgabe der Erfindung ist die Bereitstellung des Ballonkatheters, mit dem Stents in abzweigenden Gefäßen platziert und an einen im Hauptast platzierten Stent mit einer Fenestrierung angeschlossen werden können.

Diese Aufgabe wird mit einem Ballonkatheter gemäß Anspruch 1 gelöst.

Der erfindungsgemäße Ballonkatheter besteht aus einem ersten inneren Ballon und einem zweiten äußeren Ballon, der den inneren Ballon vollständig einschließt. Beide Ballone verfügen über separate Zuleitungen, so dass sie unabhängig voneinander dilatiert werden können. In der Regel wird der innere Ballon zuerst dilatiert um mit dem proximalen Ende der Ballonkonstruktion einen Stent in einem Seitenast zu platzieren, wonach der zweite äußere Ballon separat dilatiert wird, um diesen Stent im Eingangsbereich trompetenförmig aufzuweiten.

Die beiden Ballone haben einen proximalen und einen distalen Bereich. Der distale Bereich ist eher schlank gehalten. Er kann einen gleichmäßigen Durchmesser über seine Länge aufweisen, sich aber auch zum distalen Ende des Katheters hin weiter verschlanken, um eine Anpassung an sich verengende Seitenäste zu ermöglichen.

Der proximale Bereich der Ballone im erfindungsgemäßen Ballonkatheter weist eine gegenüber dem proximalen Bereich deutlich erweiterten Durchmesser auf. Insbesondere ist der Durchmesser um etwa 50 bis 100 % erweitert.

In der Regel hat der innere Ballon im proximalen Bereich einen Durchmesser (in expandiertem Zustand) von 5 bis 8 mm, der äußere Ballon von 8 bis 14 mm.

Der erfindungsgemäße Ballonkatheter kann innerhalb der Ballone eine Stufung aufweisen, die ebenfalls von einem großen Durchmesser im proximalen Bereich zu einem kleinen Durchmesser im distalen Bereich bzw. terminalen Bereich hin verläuft.

Der proximal erweiterte Bereich der Ballone weist an seinen Flanken einen relativ steilen Anstieg auf, der vorzugsweise auf beiden Seiten, d. h. der Anstieg vom Katheterschaft einerseits und der Anstieg vom proximalen Teil der Ballone andererseits, gleichmäßig ausgebildet ist. Zweckmäßigerweise beträgt der Anstieg 45 bis 75°, bezogen auf die Katheterachse. Ein steiler Anstieg der erweiterten Zone ist positiv für die trompetenförmige Aufweitung des Stents im Eingangsbereich der Abzweigung und für die Anpassung an den im Hauptast verlegten Stent.

Der erste oder innere Ballon grenzt im distalen Bereich unmittelbar an die Innenwand des zweiten Ballons und ist zweckmäßigerweise mit diesem verbunden, beispielsweise durch Verschweißen. Dies führt dazu, dass bei der Expansion des ersten Ballons eine sehr präzise Aufweitung nur im gewünschten Umfang herbeigeführt wird, eine Expansion des zweiten Ballons wirkt sich im distalen Bereich nicht aus. Im proximalen Bereich hat der erste oder innere Ballon einen deutlich kleineren Durchmesser als der äußere zweite Ballon und ist mit diesem nicht verbunden. Dies bedeutet, dass der äußere zweite Ballon individuell dilatiert werden kann und damit einen deutlich größeren Durchmesser einnehmen kann, als der erste Ballon. Dies ist positiv für die Aufweitung des Stents im Eingangsbereich der Abzweigung. Gleichzeitig ist aber der erste, innere Ballon in der ersten Phase der Aufweitung bereits geeignet, auch den Eingangsbereich des Stents in einem gewissen Maße vorzudilatieren; die "Feinjustierung" auf das gewünschte Endmaß erfolgt dann durch separate Dilatation des äußeren Zweitballons. Die umgekehrte Reihenfolge, mit einer Aufweitung des Stents im proximalen Bereich in einem ersten Schritt und der Feinjustierung durch den inneren Ballon im zweiten Schritt ist, eine Alternative dazu.

Erfindungsgemäß hat der innere Ballon im expandierten Zustand im Übergangsbereich vom proximalen zum distalen Bereich einen größeren Durchmesser als der äußere Ballon, sodass er bei der Aufweitung in einem zweiten Schritt den äußeren Ballon in diesem Bereich zusätzlich aufweitet. Der innere Ballon kann dazu in diesem Bereich gegenüber dem distalen Bereich um 25 bis 40 % im Durchmesser erweitert sein.

Der erste Ballon kann im distalen Bereich nur punktuell mit dem äußeren Ballon verschweißt sein, was eine bessere Anpassung an Unregelmäßigkeiten in der Gefäßwand ermöglicht. Auch in diesem Fall ist aber eine durchgehende Schweißnaht im Übergang vom proximalen zum distalen Bereich notwendig, um diese Expansion des zweiten Ballons auf den proximalen Bereich zu beschränken.

Gemäß einer besonderen Ausführungsform ist der erste innere Ballon im distalen Bereich wellenförmig gestaltet, so dass er punktuell an die Innenwand des äußeren Ballons heranreicht und in diesen Bereich mit ihm verschweißt ist. Auch dies ermöglicht eine sehr gute Anpassung des Stents an die Gefäßoberfläche der zu dilatierenden Abzweigung. Die Eintiefungen erlauben eine gute Druckverteilung und Anpassung an sich verengende Gefäßabschnitte.

Der erfindungsgemäße Ballonkatheter wird auf übliche Art und Weise hergestellt und gehandhabt. Auch die Materialien sind für diesen Bereich übliche Materialien. Der Unterschied zum Stand der Technik liegt allein im Design der Ballone.

Für die Ballone können übliche Materialien verwandt werden. Vorzugsweise wird für den inneren Ballon ein begrenzt aufdehnbares Material verwandt (noncompliant), etwa Polyamid 12, PET, Nylon, und für den äußeren Ballon ein gut aufdehnbares Material (compliant oder simi-compliant), etwa Silikongummi, Pebax, PA 11 oder eine Mischung aus Pebax und PA 11.

Die Erfindung wird durch die beiliegenden Zeichnungen näher erläutert. Es zeigen:
- Fig. 1:: Eine Gesamtansicht eines erfindungsgemäßen Ballonkatheters;
- Fig. 2:: eine Schnittzeichnung durch den Ballonkatheter von Figur 1;
- Fig. 3:: eine zweite Variante eines erfindungsgemäßen Ballonkatheters im Schnitt;
- Fig. 4:: eine dritte Variante eines erfindungsgemäßen Ballonkatheters; und
- Fig. 5:: einen Ballonkatheter gemäß Fig. 4 mit aufgekrimpten Stent.

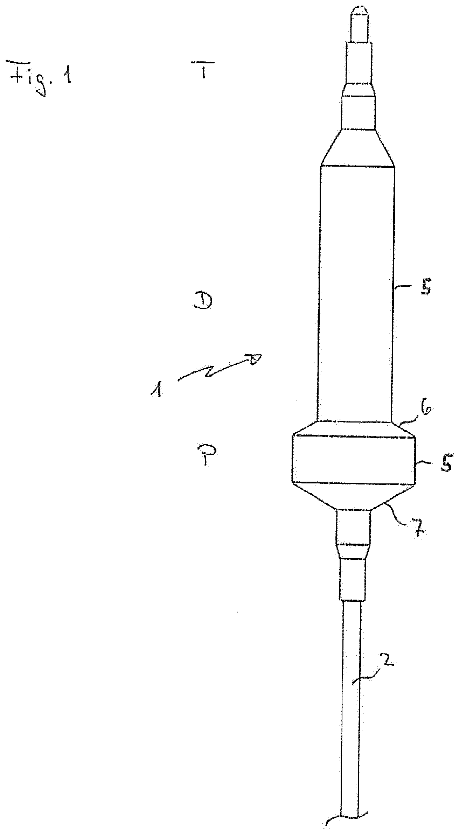

Figur 1 zeigt einen erfindungsgemäßen Ballonkatheter 1 mit dem deutlich erweiterten proximalen Bereich P mit steilen Flanken 7 zum Katheter hin und 6 zum distalen Bereich D hin, den relativ schlanken distalen Bereich D, der distal abgestuft auf den Katheterdurchmesser abnimmt. Der Katheter 2 führt, das Ballonkonstrukt hindurch und endet distal vom Ballonkonstrukt 1. Die Darstellung zeigt den Ballonkatheter in expandiertem Zustand, wobei die Kontur vom äußeren Ballon 5 bestimmt wird. Der innere Ballon (nicht dargestellt) unterstützt den äußeren Ballon 5 in dem stark aufgeweiteten Bereich P.

Für den Gebrauch ist auf den Ballonkatheter ein Stent aufgekrimpt, der durch die Expansion der Ballone aufgeweitet und in einem Blutgefäß platziert wird. Die Darstellung zeigt den Katheter 1 in expandiertem Zustand.

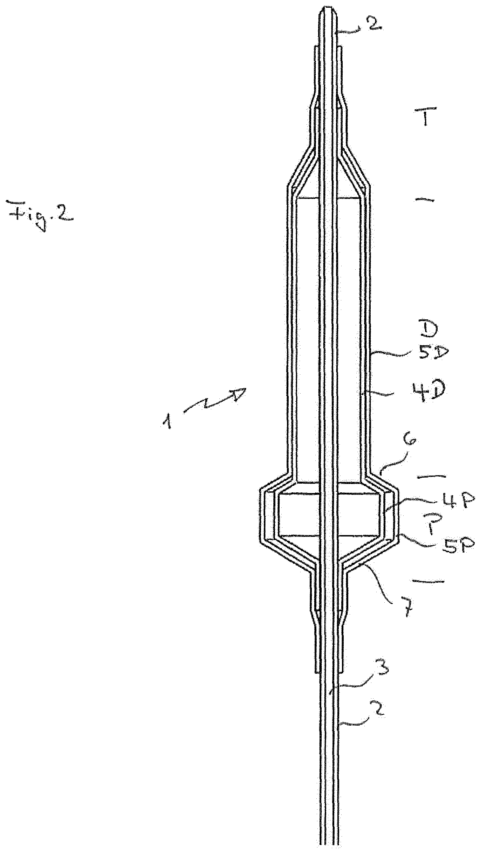

Figur 2 zeigt eine Schnittzeichnung durch den Ballonkatheter gemäß Figur 1 mit dem Katheter 2, einem freien Lumen 3 für einen Führungsdraht zum Platzieren des Katheters und dem inneren Ballon 4 sowie dem äußeren Ballon 5.

Der Doppelballon ist in den proximalen Bereich P, den distalen Bereich D und den terminalen Bereich T differenziert.

Der proximale Bereich P ist gegenüber dem distalen Bereich D deutlich erweitert, wobei der äußere Ballon 5 P einen größeren Durchmesser in diesem Bereich aufweist als der innere Ballon 4 P. Im distalen Bereich D grenzt der innere Ballon 4 D unmittelbar an die Innenwand des äußeren Ballons 5 D an und ist mit diesem verbunden. Dies bedeutet, dass bei der Dilatation der Ballone, die separat stattfinden kann, der innere Ballon grundsätzlich auch den äußeren Ballon mitnimmt und mitaufweitet. Im proximalen Bereich P ist aber der äußere Ballon 5 P über einen separaten Kanal individuell erweiterbar und weitet sich gegenüber dem inneren Ballon 4 P in diesem Bereich stärker auf, was zu einer trompetenförmigen Erweiterung und Anpassung eines platzierten Seitenaststents im Bereich der Abzweigung genutzt wird.

Im terminalen Bereich T verschlanken sich beide Ballone und schließen dichtend vor dem Ende des Katheters 2 ab. Die Kanäle, die zum Befüllen der Ballone mit dem Fluid dienen, sind konventionell und in der Zeichnung nicht dargestellt.

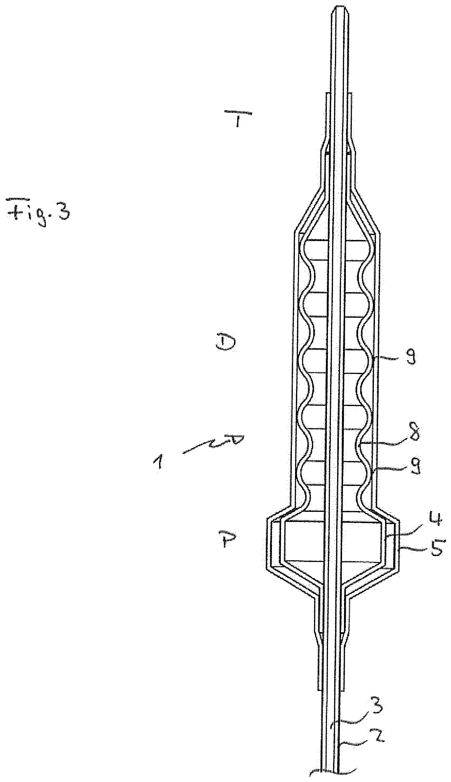

Figur 3 zeigt eine Variante des erfindungsgemäßen Doppelballons, bei der der äußere Ballon die Form hat, wie sie in Figur 1 und 2 vorgegeben ist, der innere Ballon dagegen wellenförmig ausgelegt ist, d. h. über Einschnürung 8 verfügt. Diese Einschnürungen lassen Zwischenräume zur Wandung des äußeren Ballons 5, was bedeutet, dass in diesem Bereich bei der Dilatation ein geringerer Druck auf einen Stent ausgeübt wird, als in den erweiterten Bereichen 9. An den erweiterten Bereichen 9 ist der innere Ballon 4 an den äußeren Ballon 5 angeschweißt.

Es versteht sich, dass es zahlreiche Abwandlungen im Bereich der Gestaltung des proximalen und distalen Bereichs gibt. Gemäß einer Variante ist der proximale Bereich eher kugelförmig ausgebildet. Die distalen Bereiche sind mit gleichem Durchmesser dargestellt, jedoch ist es ohne weiteres möglich, hier eine weitere Abstufung oder Verschlankung zum terminalen Ende des Katheters hin vorzusehen. So kann beispielsweise der distale Bereich über seine Länge zum terminalen Ende hin eine Reduktion des Durchmessers von 40% erfahren, wobei diese Verschlankung kontinuierlich oder stufenförmig erfolgen kann.

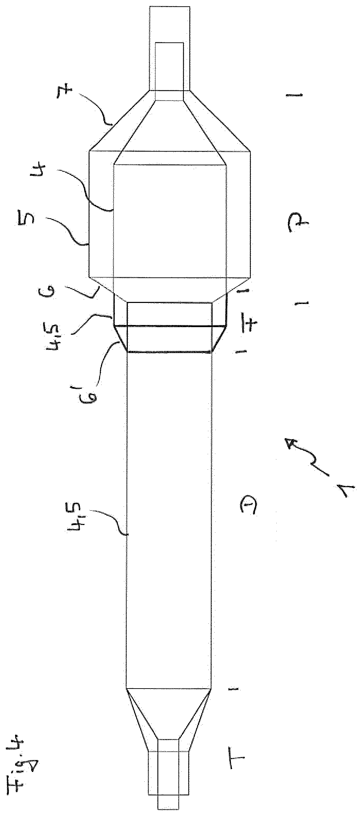

Figur 4 zeigt einen erfindungsgemäßen Doppelballon, in dem die beiden Ballone 4 und 5 voll expandiert dargestellt sind. Die Darstellung gliedert sich wiederum in einen proximalen, einen distalen und einen terminalen Bereich, wobei der proximale Bereich P gegenüber dem distalen Bereich D stark aufgeweitet ist. Dabei wird die äußere Kontur des Doppelballons im proximalen Bereich P überwiegend vom äußeren Ballon 5 bestimmt, ausgenommen der Übergangsbereich F zum distalen Bereich D, in dem der dort stärker expandierte innere Ballon 4 zu einer Aufweitung der Außenkontur im Bereich des Übergangs F führt. Der Bereich F mit der Flanke 6' wird im Wesentlichen vom expandierten inneren Ballon 4 bestimmt. Im Übrigen stimmen die Bezugszeichen mit denen von Fig. 1 bis 3 überein.

Bei der Handhabung wird ein auf den Doppelballon 1 aufgekrimpter Stent zunächst mit dem äußeren Ballon 5 expandiert. Dies führt zu einer trompetenförmigen Aufweitung des proximalen Bereichs des Stents, dort, wo der Stent in die Abzweigung aus dem Hauptgefäß eingebracht wird. In einem zweiten Schritt wird dann der innere Ballon 4 expandiert, der im Übergangsbereich F zu einer weiteren Aufweitung führt und damit den aufgekrimpten Stent in diesen Bereich an der Gefäßwand fixiert. Die mit extra starken Strichen gezeichnete Kontur im Übergangsbereich F macht deutlich, in welchem Umfang der äußere Ballon 5 durch den inneren Ballon 4 in diesen Bereich aufgeweitet wird. Es entsteht eine zweite Flanke 6' und damit ein gestufter Übergang in den distalen Bereich D.

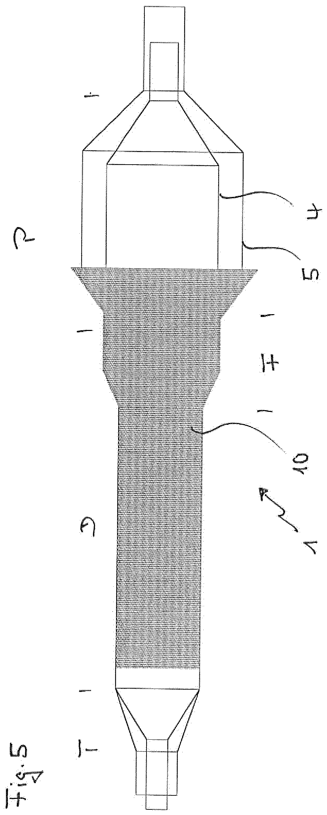

Fig. 5 zeigt den Ballonkatheter 1 mit aufgekrimpten Stent 10 der im Übergangsbereich F durch die Wirkung des inneren Ballons 4 zusätzlich gegenüber dem Bereich D aufgeweitet ist. Damit wird eine optimale Einbindung in den Eingang eines abzweigenden Gefäßes und Fixierung in diesem abzweigenden Gefäß im Eingangsbereich möglich.

## Patentansprüche

1. Ballonkatheter, insbesondere für die Aufweitung von Stents in Fenestrierungen, mit einem proximalen Bereich P, einem distalen Bereich D und einem terminalen Bereich T, einem ersten, inneren Ballon (4), einem zweiten äußeren Ballon, wobei der zweite Ballon (5) den ersten Ballon (4) vollständig umschließt, separaten Zuleitungen im Katheter zum ersten und zum zweiten Ballon (5), die es erlauben, die Ballone (4, 5) unabhängig voneinander mit Druck zu beaufschlagen, einem zentralen Lumen (3) für einen Führungsdraht, wobei im expandierten Zustand beide Ballone (4, 5) im proximalen Bereichen (P) einen gegenüber dem distalen Bereich (D) erweiterten Durchmesser aufweisen, **dadurch gekennzeichnet, dass** der innere Ballon (4) im Übergangsbereich (F) zwischen dem proximalen (P) und distalen Bereich (D) im expandierten Zustand einen größeren Durchmesser hat als der äußere Ballon (5) im distalen Bereich im expandierten Zustand, dergestalt, dass nach Aufweitung des äußeren Ballons (5) der innere Ballon (4) bei Aufweitung in einem zweiten Schritt den äußeren Ballon (5) in diesem Bereich zusätzlich aufweitet, wodurch die Außenkontur des Übergangsbereiches F im Wesentlichen vom inneren Ballon 4 bestimmt ist.

2. Ballonkatheter nach Anspruch 1, **dadurch gekennzeichnet, dass** die Ballone (4, 5) eine Stufung aufweisen.

3. Ballonkatheter nach Anspruch 1 oder 2, **dadurch gekennzeichnet, dass** der proximale Bereich (P) beider Ballone (4, 5) gegenüber dem distalen Bereich (D) um 30 % bis 100 % erweitert ist.

4. Ballonkatheter nach einem der Ansprüche 1 bis 3, **dadurch gekennzeichnet, dass** der proximale erweiterte Bereich (P) einen steilen Anstieg der Flanken (6, 7) aufweist.

## Claims

1. A balloon catheter, in particular for the expansion of stents in fenestrations, having a proximal region P, a distal region D and a terminal region T, a first, inner balloon (4), a second, outer balloon, wherein the second balloon (5) completely encloses the first balloon (4), separate leads in the catheter to the first and to the second balloon (5) allowing the balloons (4, 5) to be pressurised independently of each other, a central lumen (3) for a guide wire, wherein, in the expanded state, both balloons (4, 5) have an extended diameter in the proximal region (P) relative to the distal region (D), **characterised in that** the inner balloon (4) has a larger diameter in the transition region (F) between the proximal (P) und distal region (D) in the expanded state than the outer balloon (5) in the distal region in the expanded state, in such a way that, following expansion of the outer balloon (5), the inner balloon (4), upon expansion in a second step, additionally expands the outer balloon (5) in this region, whereby the outer contour of the transition region F is substantially determined by the inner balloon (4).

2. The balloon catheter according to claim 1, **characterised in that** the balloons (4, 5) have a gradation.

3. The balloon catheter according to claim 1 or 2, **characterised in that** the proximal region (P) of both balloons (4, 5) is extended relative to the distal region (D) by 30 % to 100 %.

4. The balloon catheter according to any one of claims 1 to 3, **characterised in that** the proximal extended region (P) has a steep rise of the flanks (6, 7).

## Revendications

1. Cathéter à ballonnet, en particulier pour l'élargissement de stents dans des fenestrations, avec une zone proximale P, une zone distale D et une zone terminale T, un premier ballonnet intérieur (4), un deuxième ballonnet extérieur, dans lequel le deuxième ballonnet (5) enferme complètement le premier ballonnet (4), des conduites d'alimentation séparées dans le cathéter vers le premier et le deuxième ballonnet (5), qui permettent de mettre les ballonnets (4, 5) sous pression indépendamment l'un de l'autre, une lumière centrale (3) pour un fil de guidage, dans lequel à l'état déployé, les deux ballonnets (4, 5) présentent dans la zone proximale (P) un diamètre dilaté par rapport à la zone distale (D), **caractérisé en ce que** le ballonnet intérieur (4) a dans la zone de transition (F) entre les zones proximale (P) et distale (D) à l'état déployé un plus grand diamètre que le ballonnet extérieur (5) dans la zone distale à l'état déployé, de sorte qu'après l'élargissement du ballonnet extérieur (5), le ballonnet intérieur (4) élargit en cas d'élargissement dans une deuxième étape le ballonnet extérieur (5) dans cette zone, le contour extérieur de la zone de transition F étant ainsi déterminé sensiblement par le ballonnet intérieur 4.

2. Cathéter à ballonnet selon la revendication 1, **caractérisé en ce que** les ballonnets (4, 5) présentent une gradation.

3. Cathéter à ballonnet selon la revendication 1 ou 2, **caractérisé en ce que** la zone proximale (P) des deux ballonnets (4, 5) est dilatée par rapport à la zone distale (D) de 30 % à 100 %.

4. Cathéter à ballonnet selon l'une quelconque des revendications 1 à 3, **caractérisé en ce que** la zone dilatée proximale (P) présente une montée raide des flancs (6, 7).
